Europäisches Patentamt

European Patent Office

Office européen des brevets

(19)

(11) Numéro de publication : **0 497 710 A2**

# (12) DEMANDE DE BREVET EUROPEEN

(21) Numéro de dépôt : 92400605.9

(22) Date de dépôt : 09.03.92

(51) Int. Cl.⁵ : **A61L 9/12, A61L 9/01**

(30) Priorité : 03.06.91 FR 9106711
03.06.91 FR 9106712

(43) Date de publication de la demande :
05.08.92 Bulletin 92/32

(84) Etats contractants désignés :
AT BE CH DE DK ES FR GB GR IT LI LU MC NL
PT SE

(71) Demandeur : SARL INTERNATIONAL
CONCEPT DIFFUSION I.C.D.
Pépinière Axone
F-69930 Saint Clément les Places (FR)

(72) Inventeur : **Veyron, Daniel Maurice**
**1512 Avenue Auguste Renoir**
**La Seyne Sur Mer (Var) (FR)**
Inventeur : **Maitre, Patrick Jean**
**Saint Clement les Places (FR)**
Inventeur : **Vernay, Laurent Nicolas**
**72, rue Marcadet**
**F-75018 Paris (FR)**

(74) Mandataire : **Rataboul, Michel Charles**
**CMR INTERNATIONAL 69, rue de Richelieu**
**F-75002 Paris (FR)**

(54) **Procédé pour diffuser un composé actif dans l'air ambiant et dispositif pour sa mise en oeuvre.**

(57)    L'invention concerne un procédé pour diffuser un composé actif dans l'air ambiant, qui consiste à former une solution diluée dudit composé actif avec un solvant volatile et non-actif, à la stabiliser, à la stocker à pression atmosphérique, à la conduire à proximité d'un flux d'air et à la disperser à pression atmosphérique dans ledit flux d'air.

L'invention concerne également un dispositif qui comprend un réservoir (10, 110) contenant une solution stable d'un composé actif, un diffuseur (40, 140) destiné à la disperser dans l'air ambiant, placé à proximité d'un flux d'air (F1-F2-F3-F4, F5-F6-F7-F8-F9-F10-F11-F12) et présentant une grande surface de contact entre ladite solution et ledit flux d'air par rapport à la quantité de solution conduite au diffuseur (40, 140) et des moyens (16-18-30, 116-118-130) pour la conduire audit diffuseur (40, 140).

L'invention s'applique plus particulièrement à la désodorisation par destruction des odeurs.

FIG.1

EP 0 497 710 A2

La présente invention concerne la diffusion dans l'air ambiant d'un composé mis sous forme liquide, dans le but de traiter cet air par exemple pour y détruire les odeurs, y introduire des parfums ou éliminer les insectes. Elle concerne plus particulièrement un procédé pour une telle diffusion et un dispositif pour sa mise en oeuvre.

On connaît un certain nombre d'appareils pour pulvériser ou diffuser un produit tels que les pulvérisateurs à pression atmosphérique, les bombes de pulvérisation sous pression, les diffuseurs "à l'air libre" par absorption d'un liquide sur un matériau poreux et les diffuseurs par effet thermique.

Cette énumération ne comprend pas la méthode de loin la plus ancienne qui consiste à faire brûler des bâtonnets d'encens, ou autre plante ou écorce parfumant l'air ambiant par combustion, compte tenu des risques de mise en oeuvre dans des lieux non adaptés tels qu'un véhicule en raison de la nécessité de la combustion pour obtenir la diffusion du parfum.

Dans la première catégorie, on peut citer parmi les systèmes les plus simples, les vaporisateurs de toilette couramment utilisés pour se parfumer ou encore les pulvérisateurs pour humidifier le linge avant repassage ou traiter les plantes. D'une manière générale, ces dispositifs comprennent d'une part, un réservoir à pression atmosphérique contenant le liquide à distribuer qui peut être, selon les cas, soit un parfum, soit de l'eau, soit une solution traitante et d'autre part, une pompe mécanique munie d'un tube plongeant dans le liquide précité qui permet, après avoir chasser l'air dans ce tube, d'aspirer le liquide et de le projeter, par action de la poussée du liquide aspiré, sous forme de fines gouttelettes par des orifices prévus à cet effet.

L'inconvénient principal de ces dispositifs est leur grande consommation en liquide.

Il a été mis au point ultérieurement, des bombes sous pression telles que les bombes dites "désodorisantes" ou les bombes insecticides dans lesquelles le produit actif est contenu dans un réservoir sous pression sous forme d'un mélange à l'état liquide avec un gaz propulseur et est pulvérisé dans l'air par l'usager avec le gaz propulseur.

Ces bombes sont d'un emploi plus agréable et plus pratique puisqu'elles nécessitent un effort moindre pour obtenir la pulvérisation en raison de la pression existant dans le réservoir et sont en outre jetables.

On retrouve néanmoins le même inconvénient que précédemment qui réside dans une consommation importante du produit auquel vient s'ajouter les problèmes de pollution et de destruction de la couche d'ozone inhérents aux gaz propulseurs (chloro-fluorocarbones). En outre, on a pu remarquer qu'en pratique, ces bombes ne présentent pas une efficacité de diffusion satisfaisante car celle-ci est localisée et seule une partie de la solution active est vaporisée, l'autre partie formant des retombées liquides.

On connaît également d'autres systèmes pour parfumer ou désodoriser un local par exemple pour lutter contre des odeurs de cuisine ou de tabac résiduelles. Dans certains cas, le diffuseur comprend un matériau poreux, de forme variable, sur lequel l'usager dépose une petite quantité de liquide parfumé stocké dans une bouteille de réserve et prévu suffisamment volatile pour pouvoir s'évaporer à température ambiante. Après dépôt, le liquide parfumé migre sur le support poreux et s'évapore au contact de l'air. Dans ce cas, le support poreux sert à réduire les quantités de liquide consommées en augmentant la surface de contact entre le liquide et l'air ambiant.

Ces dispositifs impliquent encore une consommation relativement importante de solution contenant le produit actif. Mais ils présentent surtout des inconvénients pratiques au niveau du dépôt du liquide sur le support poreux et ils sont, de ce fait, difficilement utilisables par exemple dans un véhicule automobile. Ces diffuseurs ont aussi une durée de diffusion courte et une amplitude de diffusion décroissante. On peut même dire que la diffusion est presque trop forte au début puis devient rapidement trop faible.

Dans une version plus moderne et plus pratique, les diffuseurs "à l'air libre" sont maintenant proposés aux consommateurs sous forme prête à l'emploi. Aussi, les diffuseurs vendus sous le nom commercial "SENTORETTE " ou du type plaquettes anti-moustiques sont aujourd'hui particulièrement utilisées dans les appartements comme dans les voitures. Ils comprennent un support imbibé d'une solution parfumée ou insecticide et sont rendus actifs dès l'ouverture du sachet ou du boîtier de présentation les contenant.

D'autres modèles se présentent sous la forme d'une enveloppe, souvent en carton, perforée au moins sur une partie et contenant un tissu absorbant au voisinage duquel se trouve une petite réserve de liquide. La diffusion est déclenchée en perçant la réserve de liquide à l'endroit indiqué, par exemple à l'aide d'une épingle, afin de provoquer l'écoulement du liquide sur le support absorbant; le liquide diffuse alors dans l'air ambiant par les perforations de l'enveloppe prévues à cet effet.

L'inconvénient majeur de ces systèmes réside à nouveau dans leur efficacité limitée dans le temps comme dans l'espace, efficacité qui devient rapidement insuffisante et oblige donc à un remplacement fréquent.

Par ailleurs, pour créer une ambiance parfumée, on connaît aussi des systèmes de diffusion sous l'effet de la chaleur. On peut citer dans cette catégorie, les lampes connues sous le nom commercial de "BERGER" qui consistent à provoquer la diffusion d'un liquide parfumé par évaporation sous l'action de la température.

Comme précédemment, on retrouve ici l'inconvénient d'une consommation élévée de produit. En outre, les lampes "BERGER" ne peuvent être utilisées dans une voiture en raison des conditions particulières assurant la diffusion.

Il existe par ailleurs des dispositifs qui se présentent sous la forme d'une fiche électrique munie sur sa face avant d'un logement dans lequel on introduit une petite plaquette imprégnée d'un produit, ce dispositif étant rendu actif par branchement sur une prise de courant; le produit actif diffuse alors, sous l'effet de la chaleur, par les ouvertures pratiquées dans le logement contenant la plaquette. Les dispositifs de ce type les plus répandus sont utilisés avec des plaquettes "insecticides".

Il existe des dispositifs semblables avec des recharges en forme de petites bouteilles contenant une solution du composé actif, que l'on fixe dans la prise.

Là encore, l'usager est obligé de changer souvent la plaquette ou la recharge de liquide dans la prise en raison d'une efficacité relativement limitée dans le temps. De plus, on rencontre souvent des endroits où l'on voudrait diffuser un parfum ou un insecticide mais qui ne sont pas munis de prises électriques ; ces dispositifs n'y sont alors pas utilisables.

D'une manière générale, les diffuseurs ou pulvérisateurs précités impliquent une consommation importante du liquide diffusé et une efficacité très limitée dans le temps et dans l'espace.

Il ressort de cette brève revue des systèmes existant que l'on ne dispose pas à ce jour d'un dispositif pratique, économique du point de vue consommation du liquide diffusé, d'efficacité satisfaisante et constante et qui pourrait être utilisé indifféremment dans une enceinte fixe ou mobile.

Par ailleurs, dans le cas particulier de la lutte contre les mauvaises odeurs, on ne dispose pas à ce jour de dispositifs procurant une efficacité de diffusion suffisante pour diffuser de manière satisfaisante un composé désodorisant.

On connaît par exemple le méthacrylate de lauryle pour ses propriétés désodorisantes par destruction des odeurs et non en les masquant comme la plupart des autres désodorisants connus. Cependant, ce composé dégage une mauvaise odeur lorqu'il est seul et en masse, et présente en outre une certaine instabilité qui oblige à le conserver dans des conditions particulières. Par ailleurs, il n'est actif comme destructeur d'odeurs qu'à l'état gazeux.

Par conséquent, compte tenu des ces contraintes, ce produit est actuellement utilisé sous forme de solution dans des bombes sous pression. Malheureusement, la diffusion obtenue avec une bombe sous pression est localisée autour de la bombe et, de plus, une partie du liquide vaporisé reste à l'état liquide et forme des retombées. Par suite, l'efficacité du méthacrylate de lauryle est nettement restreinte par les insuffisances de la diffusion obtenue.

L'invention a pour objectif de remédier aux inconvénients des diffuseurs connus et de fournir un procédé et un dispositif particulièrement adaptés à la diffusion d'un composé actif dans l'air ambiant d'un local comme d'un véhicule, facile à mettre en oeuvre et permettant une consommation minimale du composé actif.

Le composé actif est choisi pour ses propriétés à l'égard des substances indésirables qui se trouvent dans l'air d'une ambiance, tels que les gaz, particules ou molécules, auquel cas il s'agit d'un dépolluant.

L'invention a également pour objectif de fournir des moyens pour lutter contre les odeurs en les détruisant et non en les masquant comme tel est le cas des systèmes désodorisants actuels.

A cette fin, l'invention a pour objet un procédé pour diffuser un composé actif dans l'air ambiant, caractérisé en ce qu'il consiste :

. à former une solution diluée dudit composé actif avec un solvant volatile et non-actif,

. à stabiliser la solution obtenue,

. à stocker ladite solution à pression atmosphérique,

. à conduire ladite solution à proximité d'un flux d'air,

. à disperser à pression atmosphérique ladite solution dans ledit flux d'air.

Selon d'autres caractéristiques de l'invention :

– on récupère la solution en excès non dispersée.

– le composé actif est le méthacrylate de lauryle.

– le solvant volatile est de l'alcool éthylique.

– on forme une solution comprenant 0,1 à 5 % de méthacrylate de lauryle, 1 à 10 % d'oxystéarate de glycérine-éthylèneglycol, 50 à 95 % d'alcool éthylique, le complément à 100 % étant de l'eau.

– la solution comprend en outre une composition parfumante compatible avec le méthacrylate de lauryle.

– on fait ruisseler la solution sur un matériau plan non-absorbant placé dans le flux d'air.

– pour disperser un composé actif dans l'air ambiant d'un local, on utilise un dispositif de ventilation situé dans ledit local.

– on imprègne, au moins en partie, un matériau absorbant placé dans le flux d'air.

– on distribue la solution goutte à goutte sur le matériau absorbant.

– pour disperser un composé actif dans l'habitacle d'un véhicule notamment automobile, on utilise le flux d'air créé par un dispositif de ventilation dudit véhicule.

L'invention a également pour objet un dispositif pour diffuser un composé actif dans l'air ambiant, caractérisé en ce qu'il comprend :

. un réservoir contenant une solution stable dudit composé actif,

. un diffuseur destiné à disperser ladite solution dans l'air ambiant, ledit diffuseur étant placé à proximité d'un flux d'air et présentant une grande surface de contact entre ladite solution et ledit flux d'air par rapport à la quantité de solution conduite au diffuseur,

. des moyens pour conduire ladite solution contenant le composé actif audit diffuseur.

Selon d'autres caractéristiques de l'invention :

– le dispositif comprend en outre, des moyens de récupération de la solution en excès non dispersée.

– le diffuseur comprend d'une part, un support en matériau non absorbant placée au dessus d'une gouttière de récupération et d'autre part, un tube d'alimentation comprenant une pluralité d'orifices (50) et placé à la partie supérieure dudit support sensiblement sur toute sa largeur.

– la gouttière de récupération est en communication avec le réservoir pour recycler la solution en excès non dispersée.

– pour diffuser un composé actif dans l'air ambiant d'un local, le diffuseur est placé à proximité d'au moins un ventilateur sur le parcours du flux d'air créé par ledit ou lesdits ventilateur(s).

– le diffuseur comprend un cylindre muni, à chacune de ses extrémités, d'ouvertures destinées à canaliser le flux d'air, un revêtement absorbant placé sur la surface interne dudit cylindre et des moyens de distribution de ladite solution sur ledit revêtement absorbant.

– les moyens de distribution de la solution sur le revêtement absorbant consistent en un gicleur goutte à goutte.

– le gicleur goutte à goutte est placé à l'entrée du cylindre et sensiblement en son centre.

– le revêtement absorbant est constitué d'un matériau apte à absorber par capillarité la solution contenant le composé actif.

– pour diffuser un composé actif dans l'habitacle d'un véhicule, le diffuseur est placé à proximité d'un dispositif de ventilation dudit véhicule, sur le parcours du flux d'air créé par ledit dispositif.

– le dispositif comprend une pompe électrique destinée à aspirer la solution contenue dans le réservoir au moyen d'au moins un tuyau plongeant dans celui-ci, et à conduire ladite solution au diffuseur par un tuyau.

L'invention sera mieux comprise à la lecture de la description détaillée qui va suivre donnée à titre illustratif et non limitatif ainsi que des dessins dans lesquels :

– la figure 1 est une vue schématique en perspective d'un dispositif à ruissellement conforme à l'invention pour traiter l'air d'un local, montrant le ruissellement d'une solution d'un composé actif;

– la figure 2 est une vue schématique en perspective du dispositif selon la figure 1, en position de fonctionnement;

– la figure 3 est une vue schématique partielle en perspective d'un dispositif à absorption conforme à l'invention placé dans un véhicule automobile;

– la figure 4 est une vue schématique détaillée du diffuseur du dispositif de la figure 3;

– la figure 5 est une vue en coupe transversale du diffuseur de la figure 4;

L'invention concerne un procédé et un dispositif pour diffuser un composé actif dans l'air ambiant qui vont être tout d'abord décrits quelle que soit la solution diffusée et l'action attendue.

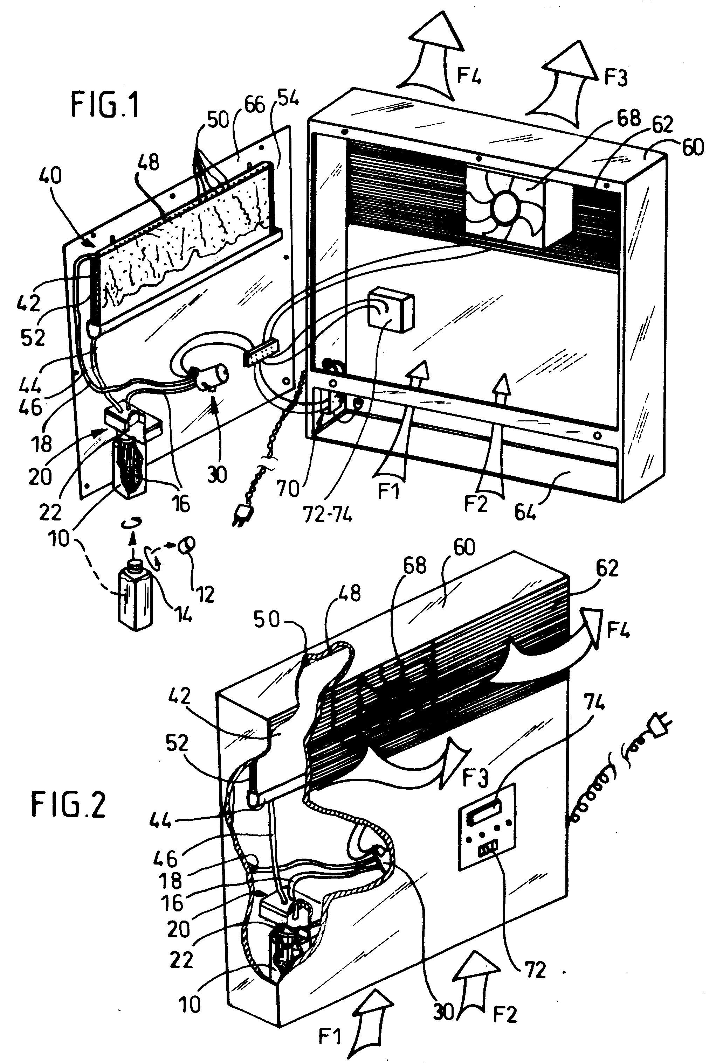

Les figures 1 et 2 représentent une forme de réalisation du dispositif selon l'invention dénommée ci-après dispositif à ruissellement.

Comme on peut le voir sur la figure 1, le dispositif comprend d'une manière générale, un réservoir 10 destiné à contenir une solution du composé actif à diffuser dans l'air ambiant et fixé sur un support 20, une pompe 30 et un diffuseur 40.

Dans l'exemple représenté, le réservoir 10 à la forme d'une bouteille et est muni d'un bouchon 12 fermé par vissage sur le goulot fileté 14. D'autres formes peuvent cependant être envisagées.

Après avoir dévissé et oté le bouchon 12, le réservoir 10 est fixé sur un support 20. Ce dernier comprend avantageusement un embout 22 correspondant au bouchon 12 et sur lequel on adapte alors par simple vissage le réservoir 10.

La pompe 30 est une pompe électrique de tout type connu et est destinée à aspirer la solution par un tube 16 plongeant dans le réservoir 10 et traversant le support 20 par un passage prévu à cet effet, puis à conduire la solution aspirée par un tube 18 au diffuseur 40.

Le diffuseur 40 comprend un support 42 constitué d'une plaque en matériau non-absorbant telle que par exemple une plaque en matière plastique rigide ou une plaque métallique. Selon une autre forme de réalisation de l'invention, le support 42 présente des propriétés de rétention de la solution active.

La plaque 42 est placée au dessus d'une gouttière de récupération 44 dont la contenance est avantageusement adaptée au débit de la pompe 30 pour éviter des débordements de la solution. On peut prévoir que la plaque 42 repose sur le fond de la gouttière 44.

La plaque 42 est munie à sa partie supérieure d'un tube d'alimentation 48 comportant une pluralité d'orifices 50 pour distribuer la solution. Le tuyau 18 est en communication avec le tube d'alimentation 48 qui reçoit ainsi la solution envoyée par la pompe 30.

La gouttière 44 est munie d'un passage pour un tuyau 46 relié au réservoir 10 et permettant de retourner audit réservoir l'excès de solution non diffusé pour le recycler.

Selon l'invention, la plaque 42 doit être placée dans le flux d'air à traiter.

Dans le cas d'un local tel qu'une pièce d'appartement, le volume d'air à traiter est important. Par suite, on crée un flux d'air convenable à l'aide, dans l'exemple décrit ici, d'un ventilateur et on place alors le diffuseur 40 dans le flux d'air créé par ledit ventilateur. Toutefois, il peut y avoir bien entendu plusieurs ventilateurs en fonction du volume à traiter et d'autres moyens pour créer un flux d'air peuvent être utilisés comme par exemple la chaleur (convecteur), une turbine, etc.

Le dispositif selon l'invention est avantageusement placé dans un boîtier 60 du type convecteur, comme illustré sur les figures 1 et 2. Le boîtier 60 comprend sur la partie supérieure de sa face avant, des ouvertures 62 et est également ouvert à sa partie inférieure 64. Un ventilateur 68 adapté au volume à traiter est placé à proximité des ouvertures 62.

Selon cette structure, la plaque 42 est fixée sur la face arrière 66 du boîtier 60 afin qu'elle se trouve au voisinage du ventilateur 68 quand le boîtier est fermé, comme schématisé sur la figure 2, sur le parcours du flux d'air engendré par ledit ventilateur. Bien entendu, les dimensions de cette plaque sont adaptées à la place disponible et à la diffusion recherchée. Elle est avantageusement la plus grande possible par rapport à la quantité de solution qu'elle doit recevoir pour obtenir une surface de contact entre la solution et l'air la plus grande possible.

Les autres éléments constitutifs du dispositif, à savoir le support 20, la pompe 30, le tube d'alimentation 48, la gouttière de récupération 44 et éventuellement les tuyaux de liaison et fils électriques, sont également fixés sur la face arrière 66 du boîtier 60 par tout moyen connu.

Le boîtier 60 est muni, sur une face latérale d'un interrupteur 70 relié à une alimentation électrique, pour commander la pompe 30 et donc la distribution de la solution contenant le composé actif. Le boîtier 60 comporte aussi un interrupteur 72 placé sur sa face avant pour contrôler le fonctionnement du ventilateur 68. Dans le cas d'un autre mode de fonctionnement, il peut n'y avoir qu'un seul interrupteur. Si désiré, on peut également prévoir la présence d'un programmateur 72 pour contrôler la mise en marche du dispositif (pompe et/ou ventilateur) et sa durée de fonctionnement.

Le boîtier 60 peut-être fixé par exemple à un mur par tout moyen connu.

Le fonctionnnement de ce dispositif est le suivant:

Avant mise en marche du dispositif, l'usager place un réservoir 10 contenant la solution désirée par vissage sur l'embout 22 du support 20.

Selon un fonctionnement manuel, l'usager appuie sur l'interrupteur 70 et déclenche ainsi la pompe 30 qui aspire alors la solution dans le réservoir 10 par le tube 16 et la conduit au tube d'alimentation 48 par le tube 18. La solution est alors distribuée en jets continus par les orifices 50 et ruisselle sur la plaque 42 en formant une couche fine sur cette plaque, comme une sorte de "rideau".

Afin d'éviter des débordements de la solution en dehors de la plaque 42 lors de son ruissellement, on prévoit des bourrelets 52 et 54 sur chacun des ses bords latéraux.

Pendant le ruissellement de la solution sur la plaque 42, l'usager commande la mise en marche du ventilateur 68 à l'aide de l'interrupteur 72 et celui-ci aspire l'air ambiant par la face inférieure ouverte 64 du boîtier 60 selon les flèches F1 et F2. Ce flux d'air vient alors au contact de la solution en couche fine et continue sur la plaque 42 qui s'évapore, pour se charger avec cette solution et la diffuser dans l'air de la pièce en ressortant du boîtier 60 par les ouvertures 62 selon les flèches F3 et F4.

L'excès de solution distribuée sur la plaque 42 et non emporté par le flux d'air, tombe dans la gouttière 44 et est retourné dans le réservoir 10.

Pour arrêter le ruissellement de la solution sur la plaque 42 et/ou le ventilateur 68, il suffit que l'usager appuie à nouveau sur l'interrupteur 70 et/ou 72.

Selon les conditions d'utilisation du dispositif, la solution diffusée et/ou les caractéristiques du support 42, le ruissellement de la solution est soit maintenu en permanence, soit réalisé par intermittence pendant le fonctionnement du ventilateur 68 qui lui est continu. Dans d'autres cas, le support 42 est choisi en fonction de la solution du composé actif de manière à pouvoir retenir une quantité suffisante de solution à sa surface ; on peut alors n'effectuer le ruissellement que pendant un temps donné avant la mise en marche du ventilateur puis arrêter la distribution de la solution et commander alors la mise en marche du ventilateur. Bien entendu, quand cela n'est plus nécessaire, on peut stopper le ventilateur et donc la diffusion.

Dans le but d'un fonctionnement automatique, on peut également programmer l'un ou l'autre des modes de fonctionnement précités suivant la destination du dispositif considéré.

Le mode de diffusion qui vient d'être décrit permet donc d'obtenir une très grande efficacité grâce à une utilisation optimale de la solution.

En outre, ce dispositif permet de n'employer que la quantité nécessaire de solution notamment grâce à la présence de moyens de récupération et de recyclage de la solution non dispersée contrairement, par exemple, aux bombes citées dans le préambule. En effet, avec ces bombes, l'usager disperse la solution sans savoir quelle quantité est réellement requise compte tenu de la pièce où il se trouve, ce qui conduit le plus souvent à des consommations excessives et inutiles. De plus, pour avoir une répartition du produit dans tout le volume de la pièce, c'est l'usager qui doit se déplacer tout en diffusant le produit. Dans tous les cas, cette répartition est faite au hasard et ne peut être homogène.

Selon l'invention, les quantités consommées sont très faibles par rapport au volume d'air traité et la répartition du produit actif est homogène puisque c'est le flux d'air lui-même qui le disperse.

On peut remarquer néanmoins que le mode de réalisation décrit précédemment ne peut être mis en oeuvre que dans le cadre d'une enceinte fixe. Du fait de la présence du diffuseur à ruissellement, il ne peut en effet être utilisé par exemple dans un véhicule automobile en raison des secousses et vibrations auxquelles il est soumis lorsqu'il est en mouvement.

Par ailleurs, il faut noter que le volume d'air à traiter dans l'habitacle d'un véhicule est moins important que dans un local ; aussi, la surface de contact entre la solution active et l'air peut donc être moins importante tout en restant grande par rapport à la quantité de solution distribuée.

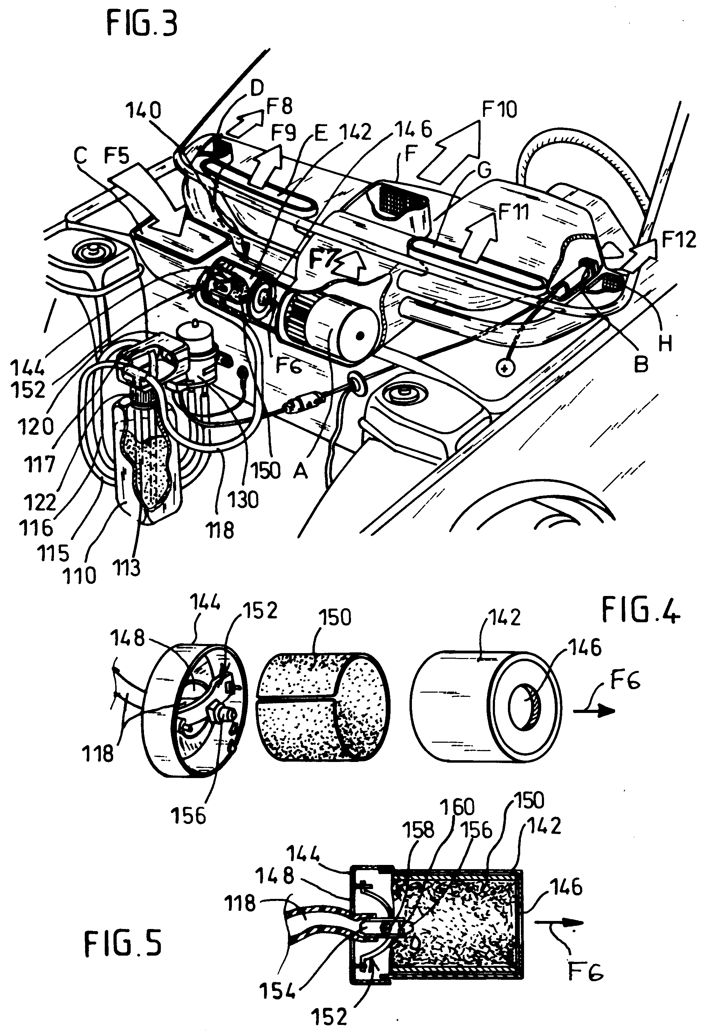

Les figures 3 à 5 illustrent une forme de réalisation du dispositif selon l'invention adaptée à une utilisation dans une enceinte en mouvement.

Comme on peut le voir sur la figure 3, le dispositif comprend un certain nombre d'éléments analogues à ceux décrits dans le premier mode de réalisation donné ci-avant.

En effet, le dispositif comprend un réservoir 110 destiné à contenir la solution du composé actif désiré, fixé sur un support 120 par vissage sur un embout 122.

Une pompe 130 est prévue pour aspirer la solution contenue dans le réservoir 110 et la conduire au diffuseur 140 par des tubes respectivement 116 et 118, par exemple en matière synthétique flexible, bien entendu compatible avec la solution à disperser.

La différence essentielle du second exemple de réalisation donné ici se situe au niveau du diffuseur 140. En effet, il ne s'agit plus d'un diffuseur à ruissellement mais d'un diffuseur à absorption.

La figure 4 représente plus en détails le diffuseur 140 et on voit qu'il est constitué d'un corps cylindrique formé de deux parties 142 et 144 emboîtées l'une dans l'autre et muni à ses extrémités d'ouvertures 146 et 148 pour le passage de l'air.

Un revêtement 150 en matière absorbante est placé à l'intérieur du cylindre 142-144 de manière à recouvrir sa surface interne. Dans l'exemple donné ici, il s'agit d'un revêtement du type éponge qui est d'abord introduit dans la partie 142 du cylindre, sur laquelle est ensuite adaptée l'autre partie 144. Dans d'autres cas, on peut employer un tissu ou un feutre présentant des propriétés d'absorption par capillarité.

Un gicleur 152 est destiné à distribuer goutte à goutte la solution sur le revêtement 150. Comme on peut mieux le voir sur la figure 5, le gicleur 152 comporte d'une part, du côté extérieur au cylindre, un embout 154 sur lequel vient s'adapter un tuyau 118 pour l'alimentation en solution et d'autre part, du côté interne au cylindre, un embout 156 dans lequel est logée une bille 158 destinée à coopérer avec un siège 160. Le gicleur 152 est avantageusement placé à l'entrée du cylindre 142-144 selon son axe central.

Selon l'invention, le diffuseur 140 doit être positionné sur le parcours d'un flux d'air. Dans le cas considéré, s'agissant de traiter l'air de l'habitacle d'un véhicule automobile, on peut avantageusement utiliser le dispositif de ventilation de ce véhicule ou le dispositif d'air conditionné, s'il en est muni.

La figure 3 illustre un cas où on utilise le dispositif de ventilation du véhicule. Le dispositif selon l'invention est inséré dans la partie "moteur" du véhicule, sous le capot avant, et le diffuseur 140 est placé à proximité de la turbine de ventilation A de sorte que le corps cylindrique 142-144 soit, en cours de fonctionnement, traversé de part en part par le flux d'air.

Par ailleurs, on prévoit dans l'habitacle, par exemple sur le tableau de bord, un interrupeur B permettant de controler le dispositif.

Le fonctionnement de ce dispositif à absorption est le suivant :

Lorsque par exemple, le conducteur du véhicule souhaite traiter l'air de l'habitacle où il se trouve, il déclenche le fonctionnement du dispositif par l'interrupteur B. Celui-ci commande la pompe 130 qui débute alors l'aspiration de la solution active contenue dans le réservoir 110 par le tuyau 116 et conduit ensuite la solution aspirée au diffuseur 140 par le tuyau 118.

Dans l'exemple donné, on a prévu avantageusement un tube en T 117 sur lequel sont adaptés un tuyau 115 venant de la pompe 130, le tuyau 118 servant à alimenter le diffuseur 140 et un tube 113 plongeant dans le réservoir 110. Ce tube en T 117 permet d'éviter un éventuel engorgement de la pompe 130 qui serait dû à une puissance de pompage excessive et de recycler l'excès de solution pompé.

La solution pompée arrive ensuite au gicleur 152 par le tuyau 118 et vient plaquer la bille 158 sur le siège 160.

La bille 158 et le siège 160 sont calibrés afin de laisser un passage résiduel lorsque la bille est plaquée sur son siège, ce passage permettant d'obtenir une distribution goutte à goutte de la solution sur le revêtement absorbant 150. Le gicleur 152 permet donc de contrôler le débit de la distribution. La solution distribuée est alors absorbée par capillarité par ledit revêtement 150.

Par mesure de sécurité, les ouvertures 146 et 148 sont avantageusement pratiquées selon un axe sensiblement parallèle à l'axe du cylindre et au dessus de ce dernier de sorte qu'un éventuel débordement de la solution résultant d'un débit trop important puisse être évité ou pour le moins limité.

Compte tenu du positionnement du cylindre diffuseur 142-144, l'air aspiré par la turbine de ventilation A selon la flèche F5 pénètre dans le conduit C puis est canalisé dans le cylindre 142-144 qu'il traverse par les ouvertures 148 et 146.

En passant dans le cylindre 142-144, l'air vient au contact de la solution absorbée dans le revêtement 150 et se charge avec cette solution.

Le flux d'air chargé en solution ressort du cylindre 142-144 selon la flèche F6 et est finalement dispersé dans l'habitacle par l'ensemble des bouches de ventilation prévues D, E, F, G, H, ou seulement celles choisies, selon les flèches F7, puis F8, F9, F10, F11 et F12.

Lorsque la dispersion de la solution n'est plus souhaitée ou nécessaire, l'usager actionne à nouveau l'interrupteur B pour stopper le fonctionnement de la pompe 130. Il n'y a alors plus de pression qui s'exerce sur la bille 148 et celle-ci s'écarte donc du siège 160.

La forme fermée et cylindrique du diffuseur 140 est particulièrement avantageuse car elle procure une grande surface de contact entre l'air et la solution absorbée. En outre, la solution se trouve concentrée autour du flux d'air traversant le cylindre, ce qui améliore encore le contact entre le flux d'air et la solution et donc l'efficacité de la diffusion.

Par ailleurs, le dispositif selon l'invention permet de réduire la consommation en solution active grâce au contrôle de la distribution de la solution, qui est suffisamment lente et régulière pour permettre une absorption satisfaisante de ladite solution sur le support absorbant.

Selon la vitesse d'évaporation de la solution, le diffuseur reste efficace encore un certain temps après arrêt de la distribution de la solution en raison de son absorption et de la forme cylindrique du diffuseur. Dans ce cas, l'air qui traverse le diffuseur , après arrêt du gicleur, peut se charger en solution active comme explicité précédemment.

Le dispositif selon l'invention est particulièrement bien adapté pour la dispersion de composés actifs notamment dans le but de désodoriser l'air ambiant.

A cette fin, il est donné dans ce qui suit, un exemple d'utilisation avec une composition désodorisante et éventuellement parfumante.

Cette composition se distingue des solutions utilisées jusqu'à présent dans les diffuseurs ou pulvérisateurs courants, par le fait qu'elle permet de détruire les odeurs et non de les masquer d'une manière plus ou moins efficace et agréable, comme tel est le cas des diffuseurs désodorisants connus.

La composition utilisée dans le cadre de l'invention consiste en une solution stabilisée de méthacrylate de lauryle dans un solvant volatile.

Selon les conditions de son utilisation, la solution de méthacrylate de lauryle doit répondre à certaines exigences de stabilité et de sécurité. En effet, si l'on considère par exemple le cas d'un dispositif implanté dans un véhicule automobile, dans le compartiment moteur, la solution doit être ininflammable. De même, cette condition doit être satisfaite dans le cadre d'un usage domestique, pour traiter un local.

On choisit avantageusement comme solvant, un mélange d'alcool éthylique et d'eau.

Bien entendu, la formulation de la composition dépend également des propriétés physiques du méthacrylate de lauryle et notamment de sa faible solubilité dans les solvants, en particulier dans un degré d'alcool assez bas nécessaire pour satisfaire aux exigences précitées. Aussi, la solution comprend un produit solubilisant et émulsifiant.

Par ailleurs, la solution contient, si désiré, une composition parfumante pour parfumer l'air ambiant parallèlement à la destruction des odeurs nauséabondes.

Du fait des propriétés désodorisantes par destruction des odeurs du méthacrylate de lauryle, la composition parfumante est choisie de manière à être compatible avec celui-ci. On entend par compatible, une composition parfumante susceptible de co-exister avec le méthacrylate de lauryle lors de son stockage ou de sa distribution, sous forme de solution, comme après diffusion; la composition parfumante est sélectionnée afin de ne pas être détruite en même temps que les odeurs indésirables.

La composition selon l'invention comprend :
– 0,1 à 5 % de méthacrylate de lauryle,
– 1 à 10 % d'un produit émulgateur tel que l'oxystéarate de glycérine-polyéthylèneglycol vendu sous le nom commercial de "CREMOPHOR RH" par la société BASF, dont le siège social est situé 49 avenue Georges Pompidou, 92 593 Levallois Perret (France).
– 50 à 95 % d'alcool éthylique,
– 0 à 10 % d'une composition parfumante,
– complément en eau.

Dans le cas de la première forme de réalisation du dispositif selon l'invention, la solution décrite ci-dessus est placée dans un réservoir 10. Le flux d'air créé au moyen du ventilateur 68 se charge en composition à base de méthacrylate de lauryle au niveau de la plaque 52 puis se répand dans la pièce en dispersant le composé actif dans le local, par les ouvertures 62 selon les flèches F3 et F4.

Dans l'exemple décrit ici, du fait notamment de la grande efficacité de la composition, de sa rétention sur la plaque 42 et de sa vitesse d'évaporation, il n'est pas nécessaire de faire ruisseler en permanence la solution sur la plaque 42. Il suffit de déclencher la pompe 30 par intermittence au moyen de l'interrupteur 70, à des intervalles de temps déterminés et par exemple programmés au moyen du programmateur 74 ou, plus simplement, lorsque l'activité recherchée n'appartaît plus suffisante.

On peut remarquer que selon l'invention, on a une évaporation quasiment complète de la composition contrairement au cas des bombes sous pression dans lequel une partie plus ou moins importante de solution reste à l'état liquide et forme des retombées, ce qui conduit à une efficacité moindre. En outre, du fait des gaz propulseurs, la présence d'eau est exclue alors qu'elle est permise dans le cadre de l'invention ; le degré d'inflammabilité de la solution selon l'invention étant ainsi réduit, le dispositif de diffusion peut être placé dans les lieux les plus favorables au niveau de l'efficacité.

Le méthacrylate de lauryle se trouve présent dans l'air ambiant en faible quantité et ne génère pas de mauvaise odeur dans ces conditions. Cette faible quantité est toutefois suffisante pour être efficace et détruire les odeurs règnant dans toute la pièce grâce à l'efficacité de la diffusion. Après traitement, aucune odeur n'est perceptible.

Dans le cas où une composition parfumante est introduite dans la composition dispersée, l'air ambiant est parfumé lors de la diffusion puis le parfum s'atténue peu à peu pour disparaître complètement, comme cela se produit habituellement. En effet, compte tenu de sa formulation, l'activité de la composition parfumante n'est pas influencée par celle du méthacrylate de lauryle et chacun de ces constituants agit indépendamment.

L'invention s'adapte également à des enceintes de dimensions réduites : petites pièces, placards et armoires, boîtes. On peut donc utiliser l'invention pour détruire les odeurs ou dépolluer des atmosphères relativement confinées: locaux contenant des réceptacles à ordures, placards à chaussures, armoires contenant des objets malodorants, niches à chien, endroits où se trouvent des litières pour chats etc.

On peut soit réaliser des dispositifs indépendants que l'on met en place à la demande, soit fabriquer des ensembles incorporant à la fois un volume utile et un dispositif.

De toutes façons, chaque dispositif comprend un ventilateur, un réservoir de produit et un diffuseur qui, selon le volume en cause est du type à ruissellement, à goutte-à-goutte ou à distributeur programmé.

Dans le cas du second mode de réalisation du dispositif selon l'invention, la solution à base de méthacrylate de lauryle est introduite dans le réservoir 110. Lorsque l'un des passagers du véhicule fume et souhaite ne pas indisposer les autres passagers par les odeurs de tabac, il suffit qu'il appuie, par intermittence, sur l'interrupteur 70 pour actionner de temps en temps la pompe 130. Celle-ci alimente alors le diffuseur 140 et la solution à base de méthacrylate de lauryle est distribuée goutte à goutte par le gicleur 152 sur le revêtement absorbant 150. Lorsque le revêtement 150 est imbibé, au moins en partie, les passagers peuvent ouvrir l'ensemble des bouches de ventilation D, E, F, G, H ou seulement certaines d'entre elles et le flux d'air qui a traversé le cylindre 142-144 selon les flèches F5, F6 et qui est donc chargé en composition active, se disperse ensuite dans l'habitacle en diffusant en même temps le méthacrylate de lauryle et éventuellement un parfum. Le méthacrylate de lauryle peut alors agir et détruire les odeurs de tabac.

Il n'est pas utile de faire fonctionner le dispositif en permanence, compte tenu de la forme cylindrique du diffuseur 140 qui "retient" la composition évaporée dans son enceinte et de la grande efficacité dans le temps ainsi obtenue. Seule une quantité très faible de méthacrylate de lauryle est nécessaire.

Il faut noter également l'efficacité très rapide de cette composition à base de méthacrylate de lauryle car même si le passager continue de fumer, l'odeur de cigarette ou de cigare est détruite quasiment au fur et à mesure et n'est pas perceptible.

De même, lorsque l'on rentre dans un véhicule qui a été occupé auparavant par un fumeur et traité à l'aide de la solution à base de méthacrylate de lauryle, on constate que l'odeur de tabac n'est pas réapparue. Ceci montre bien que la solution utilisée détruit les odeurs et ne se contente pas de les masquer, comme avec les désodorisants disponibles à ce jour. En effet, avec ces-derniers, une fois le parfum disparu, les odeurs nauséabondes sont à nouveau perceptibles.

Selon les objectifs recherchés, le dispositif selon l'invention peut servir à la dispersion dans l'air ambiant d'autres composés actifs comme par exemple des insecticides ou des compositions pour simplement éloigner les moustiques, des solutions aseptisantes, etc.

## Revendications

1/ Procédé pour diffuser un composé actif dans l'air ambiant, caractérisé en ce qu'il consiste :
. à former une solution diluée dudit composé actif avec un solvant volatile et non-actif,
. à stabiliser la solution obtenue,
. à stocker ladite solution à pression atmosphérique,
. à conduire ladite solution à proximité d'un flux d'air,
. à disperser à pression atmosphérique ladite solution dans ledit flux d'air.

2/ Procédé selon la revendication 1, caractérisé en ce que l'on récupère la solution en excès non dispersée.

3/ Procédé selon la revendication 1, caractérisé en ce que le composé actif est le méthacrylate de lauryle.

4/ Procédé selon la revendication 3, caractérisé en ce que le solvant volatile est de l'alcool éthylique.

5/ Procédé selon la revendication 4, caractérisé en ce que l'on forme une solution comprenant 0,1 à 5 % de méthacrylate de lauryle, 1 à 10 % d'oxystéarate de glycérine-éthylèneglycol, 50 à 95 % d'alcool éthylique, le complément à 100 % étant de l'eau.

6/ Procédé selon l'une quelconque des revendications 3 à 5, caractérisé en ce que la solution comprend en outre une composition parfumante compatible avec le méthacrylate de lauryle.

7/ Procédé selon la revendication 1, caractérisé en ce que l'on fait ruisseler la solution sur un matériau plan non-absorbant placé dans le flux d'air.

8/ Procédé selon la revendication 7, caractérisé en ce que pour disperser un composé actif dans l'air ambiant d'un local, on utilise un dispositif de ventilation situé dans ledit local.

9/ Procédé selon la revendication 1, caractérisé en ce que l'on imprègne, au moins en partie, un matériau absorbant placé dans le flux d'air.

10/ Procédé selon la revendication 9, caractérisé en ce que l'on distribue la solution goutte à goutte sur le matériau absorbant.

11/ Procédé selon la revendication 9, caractérisé en ce que pour disperser un composé actif dans l'habitacle d'un véhicule notamment automobile, on utilise le flux d'air créé par un dispositif de ventilation dudit véhicule.

12/ Dispositif pour diffuser un composé actif dans l'air ambiant, caractérisé en ce qu'il comprend :

. un réservoir (10, 110) contenant une solution stable dudit composé actif,

. un diffuseur (40, 140) destiné à disperser ladite solution dans l'air ambiant, ledit diffuseur étant placé à proximité d'un flux d'air (F1-F2-F3-F4, F5-F6-F7-F8-F9-F10-F11-F12) et présentant une grande surface de contact entre ladite solution et ledit flux d'air par rapport à la quantité de solution conduite au diffuseur (40, 140),

. des moyens (16-18-30, 116-118-130) pour conduire ladite solution contenant le composé actif audit diffuseur (40, 140).

13/ Dispositif selon la revendication 12, caractérisé en ce qu'il comprend en outre, des moyens de récupération (44-46) de la solution en excès non dispersée.

14/ Dispositif selon la revendication 12, caractérisé en ce que le diffuseur comprend d'une part, un support (42) en matériau non absorbant placée au dessus d'une gouttière de récupération (44) et d'autre part, un tube d'alimentation (48) comprenant une pluralité d'orifices (50) et placé à la partie supérieure dudit support (42) sensiblement sur toute sa largeur.

15/ Dispositif selon la revendication 12, caractérisé en ce que la gouttière de récuperation (44) est en communication avec le réservoir (10) pour recycler la solution en excès non dispersée.

16/ Dispositif selon l'une quelconque des revendications 12 à 15, caractérisé en ce que pour diffuser un composé actif dans l'air ambiant d'un local, le diffuseur (40) est placé à proximité d'au moins un ventilateur (68) sur le parcours du flux d'air (F1-F2-F3-F4) créé par ledit ou lesdits ventilateur(s).

17/ Dispositif selon la revendication 12, caractérisé en ce que le diffuseur comprend un cylindre (142-144) muni, à chacune de ses extrémités, d'ouvertures (146-148) destinées à canaliser le flux d'air (F5-F6), un revêtement absorbant (150) placé sur la surface interne dudit cylindre (142-144) et des moyens de distribution (152) de ladite solution sur ledit revêtement absorbant (150).

18/ Dispositif selon la revendication 17, caractérisé en ce que les moyens de distribution de la solution sur le revêtement absorbant (150) consistent en un gicleur goutte à goutte (152).

19/ Dispositif selon la revendication 18, caracté"risé e, ce que le gicleur goutte à goutte (152) est placé à l'entrée du cylindre (142-144) et sensiblement en son centre.

20/ Dispositif selon la revendication 17, caractérisé en ce que le revêtement absorbant (150) est constitué d'un matériau apte à absorber par capillarité la solution contenant le composé actif.

21/ Dispositif selon l'une quelconque des revendications 17 à 20, caractérisé en ce que pour diffuser un composé actif dans l'habitacle d'un véhicule, le diffuseur (140) est placé à proximité d'un dispositif de ventilation (A-C-D-E-F-G-H) dudit véhicule, sur le parcours du flux d'air (F5-F6-F7-F8-F9-F10-F11-F12) créé par ledit dispositif.

22/ Dispositif selon la revendication 12, caractérisé en ce qu'il comprend une pompe électrique (30, 130) destinée à aspirer la solution contenue dans le réservoir (10, 110) au moyen d'au moins un tuyau (16, 116) plongeant dans celui-ci, et à conduire ladite solution au diffuseur (40, 140) par un tuyau (18, 118).

FIG.1

FIG.2

FIG.3

FIG.4

FIG.5